# DEMANDE DE BREVET EUROPEEN

(11) **EP 0 590 407 A1**
(43) Date de publication de la demande: **06.04.1994**
(21) Numéro de dépôt: 93114820.9
(22) Date de dépôt: 15.09.1993
(51) Int. Cl.: A61M 25/01

(54) **Support de guidage pour cathéter formé notamment d'une gaine roulée multibrins**

(30) Priorité: 29.09.1992 FR 9211733
(71) Demandeur: Nivarox-FAR S.A., CH-2400 Le Locle (CH)
(72) Inventeur: Guy, Grenouillet, F-25130 Villers-Le-Lac (FR)
(74) Mandataire: de Montmollin, Henri

(57) **Abrégé**

L'invention concerne un support de guidage (1) pour cathéter comprenant un fil (2) ayant une partie proximale (6) de section sensiblement constante et une partie distale (8) effilée, et une gaine (4) de protection souple formée d'un ressort hélicoïdal s'étendant au moins autour de la longueur de la partie distale (8) effilée, le fil (2) étant rendu solidaire de la gaine (4) par au moins l'extrémité libre de sa partie proximale au moyen d'une première soudure (10) dans une partie proximale (12) de la gaine, l'extrémité libre (14) de la partie distale de la gaine (4) étant munie d'une deuxième soudure (16) formant un bout arrondi et qui est caractérisé en ce que le ressort qui forme la gaine comprend un premier brin (18) roulé avec un premier pas pour former une série de premières spires (S18) et au moins un deuxième brin (20, 22, 24) roulé avec un deuxième pas d'une valeur sensiblement supérieure à celle du premier pas pour former une série de deuxièmes spires (S20, S22, S24), de manière que les premières spires (S18) exercent une force de compression sur les deuxièmes spires (S20, S22, S24)

## Description

L'invention concerne les supports de guidage en général et plus particulièrement un support de guidage formé d'une gaine roulée multibrins et destiné à être introduit dans des vaisseaux sanguins de faible diamètre pour faciliter l'introduction d'un cathéter.

Ce type de gaines trouve de nombreuses applications notamment dans le cadre de la chirurgie cardiovasculaire comme, par exemple, lors d'une angioplastie coronarienne, pendant laquelle on introduit un cathéter dans une région rétrécie de l'artère coronaire du patient et l'on gonfle ce cathéter pour dilater cette région rétrécie.

On connait déjà du document US 4 548 206 un support de guidage pour cathéter destiné à être introduit par voie endoveineuse dans le corps humain.

Selon ce document, le support de guidage comprend un fil ou stylet comportant une partie proximale de diamètre constant et une région distale effilée. Une gaine formée d'un ressort hélicoïdal monofil présentant un diamètre intérieur sensiblement égal au diamètre de la partie proximale du fil est enfilée sur le fil. L'extrémité proximale du fil est fixée à l'extrémité proximale du ressort à l'aide d'une brasure et l'extrémité distale du fil est laissée libre dans la région distale du ressort. Enfin , le support de guidage comprend un lacet de sécurité formé d'un fil de faible diamètre qui s'étend sur toute la longueur du ressort à l'intérieur de celui-ci, et qui est fixé à ses deux extrémités également par une brasure. Ce lacet de sécurité a pour fonction de maintenir la cohésion des spires du ressort et d'éviter la dislocation de celui-ci lorsqu'il est soumis à une traction.

Toutefois, compte tenu du très faible diamètre de ces support, de l'ordre de 0,5 mm, et de leur longueur relativement importante (jusqu'à 2 m), ces supports présentent une grande souplesse qui les rend fragiles et délicats à manipuler, à stocker ou à transporter.

En effet, on s'est aperçu qu'au cours des opérations de manipulation et de transport des gaines, un grand nombres de celles-ci subissaient des déformations et notamment des déboitages de spires, qui les rendent impropres à une quelconque utilisation.

En effet un tel déboitage forme une surépaisseur qui gêne, voire bloque le glissement du cathéter sur le support.

Par ailleurs, la nécessité de la préparation des lacets de sécurité qui doivent être successivement tréfilés à un diamètre convenable afin d'être ensuite laminés, redressés, découpés à longueur, ébavurés, puis lavés, avant d'être introduits manuellement dans les gaines et d'être soudés rend la fabrication coûteuse et fastidieuse.

Il faut également noter que la section du lacet de sécurité est très faible (1,4x10⁻²mm) et que la résistance de ses attaches est faible. On comprend donc aisément qu'une manipulation maladroite du support peut conduire aisément à une traction sur le lacet entraînant sa rupture. Une telle rupture peut provoquer le débobinage du ressort qui si cela se produit dans une veine d'un patient peut causer de graves complication notamment lors de son extraction.

L'invention a donc pour but principal de remédier aux inconvénients de l'art antérieur susmentionné en fournissant un support de guidage présentant une meilleure fiabilité et dont la fabrication est simple et peu couteuse.

A cet effet l'invention a pour objet un support de guidage pour cathéter comprenant un fil ayant une partie proximale de section sensiblement constante et une partie distale effilée, et une gaine de protection souple formée d'un ressort hélicoïdal s'étendant au moins autour de la longueur de la partie distale effilée, le fil étant rendu solidaire de la gaine par au moins l'extrémité libre de sa partie proximale au moyen d'une première soudure dans une partie proximale de la gaine, l'extrémité libre de la partie distale de la gaine étant munie d'une deuxième soudure formant un bout arrondi, caractérisé en ce que le ressort formant la gaine comprend, sur toute sa longueur, un premier brin roulé avec un premier pas pour former une série de premières spires et au moins un deuxième brin roulé avec un deuxième pas d'une valeur sensiblement supérieure à celle du premier pas pour former une série de deuxièmes spires, de manière que les premières spires exercent une force de compression sur les deuxièmes spires.

Ainsi, on augmente la cohésion des spires du ressort. La cohésion des spires étant ainsi renforcée on diminue le risque de déboitage des spires et on s'affranchit du lacet de sécurité et des inconvénients qui l'accompagnent.

Selon une caractéristique avantageuse de l'invention, le ressort comprend quatre brins.

L'utilisation de quatre brins s'est avérée constituer un bon compromis prenant en compte la complexité des machines utilisées pour réaliser la gaine, la cohésion des spires obtenue et le temps roulage des gaines qui est diminué par rapport au temps nécessaire pour réaliser une gaine comprenant un ressort monofil.

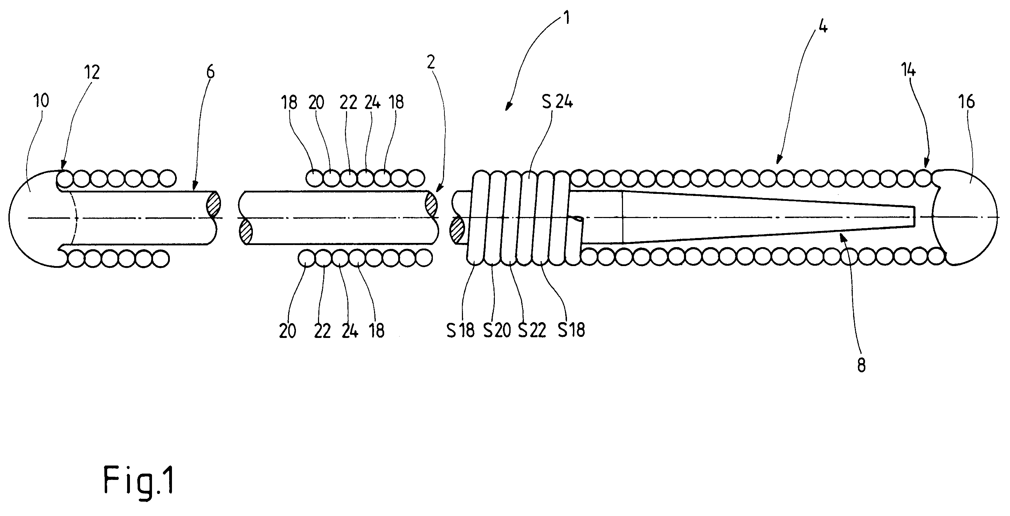

D'autres caractéristiques et avantages de l'invention apparaîtront plus clairement à la lecture de la description suivante d'un mode de réalisation donné à titre purement illustratif et non limitatif d'un support de guidage selon l'invention, cette description étant faite en liaison avec la figure 1 unique représentant une coupe fragmentée d'un mode de réalisation du support de guidage selon l'invention.

En se référant à cette figure 1, on voit un support de guidage pour cathéter selon l'invention désigné par la référence générale 1. Un tel support de guidage est couramment utilisé en chirurgie lors de la mise en oeuvre de procédés d'angioplastie, notamment pour faciliter l'introduction et le positionnement d'un cathéter tel qu'un cathéter d'exploration, de traitement ou analogue dans le système cardiovasculaire d'un patient. Pour la mise en place du cathéter il est donc nécessaire d'introduire, dans un premier temps, le support de guidage jusqu'à la zone à traiter du système cardiovasculaire puis, dans un deuxième temps de faire glisser le cathéter désiré jusqu'à la zone à traiter.

Comme cela ressort clairement de la figure 1, le support de guidage 1 comprend un fil 2, ou stylet, formant l'âme du support, sur lequel est enfilée une gaine de protection 4 solidaire de ce fil.

Le fil 2, réalisé de préférence en acier inoxydable, comprend une partie proximale 6 de section sensiblement constante et une partie distale 8 effilée.

La partie proximale 6 présente une forme générale cylindrique et la partie distale 8 présente une forme tronconique. On notera à ce propos que la partie distale 8 peut présenter toute autre forme en fonction par exemple du degré de souplesse requis pour l'application dans laquelle le support 1 doit être utilisé.

A titre d'exemple et pour fixer les idées, le diamètre de la partie 6 à section constante du fil 2 est de l'ordre de 0,3 mm et le diamètre de l'extrémité de la partie distale du fil 2 est de l'ordre de 0,1 mm.

La gaine de protection 4 est formée par un ressort hélicoïdal à spires jointives. Dans l'exemple du support 1 représenté, la gaine 4 s'étend autour du fil 2 et sur toute sa longueur. On remarquera aussi que le diamètre intérieur de la gaine 4 est sensiblement égal au diamètre de la partie proximale 6 du fil 2.

La gaine 4 est rendue solidaire du fil 2 au moyen d'une première soudure 10 qui relie sa première extrémité 12, située dans sa partie proximale, à l'extrémité libre de la partie proximale 6 du fil 2. La deuxième extrémité libre 14 de la gaine 4 est munie d'une deuxième soudure 16 qui forme un bout arrondi dont le diamètre est de préférence sensiblement égal à celui de la gaine 4. Ainsi, de par la forme arrondie et la dimension de cette soudure 16, le risque de lésion de la paroi interne d'une veine lors de l'introduction dans celle-ci d'un support 1 est fortement diminué et cette introduction est aussi facilité.

Quant à l'extrémité distale 8 du fil 2, elle se trouve libre à une certaine distance de la deuxième extrémité 14 de la gaine. Cette distance peut bien entendu varier en fonction de la souplesse que l'on désire obtenir en bout du support 1 et dans une autre forme d'éxecution, l'extrémité distale 8 du fil 2 peut être fixée à la soudure 16.

On notera que les soudures se font de façon avantageuse sans apport de matière.

Conformément à l'invention, le ressort formant la gaine 4 comprend un premier brin 18 roulé avec un premier pas P1 pour former une série de premières spires S18 et trois autres brins 20, 22, et 24 roulés respectivement avec un deuxième pas P2 d'une valeur sensiblement supérieure à celle du premier pas P1 pour former trois séries de deuxièmes spires S20 S22, et S24. Le pas est bien entendu défini par la distance en deux spires successives formées par un même brin lorsque ce brin est à l'état libre. Ainsi, si les spires S22, S24, et S26 était libres, elles auraient un pas P2 plus grand que celui de la spire S20 de manière que cette dernière comprime les autres et maintien une cohésion des spires entre elles.

Par rouler le premier brin et les trois autres brins respectivement avec un premier pas P1 et un deuxième pas P2, on comprendra que la machine roulant le premier et les trois autres brins est réglée de façon appropriée pour conformer le premier brin et les trois autres brins avec le pas P1 respectivement avec le pas P2.

De par cette structure les spires successives S22, S24, S26 respectivement des trois autres brins 22, 24, 26 sont maintenues comprimées régulièrement par deux spires S20 successives du premier brin 20 si bien que la raideur du ressort formant la gaine 4 et augmentée est par là même la cohésion de ses spires améliorée.

Bien entendu le résultat du roulage du ressort multibrins formant la gaine conduit à la fabrication d'un ressort à spires jointives, les pas P1 et P2 représentant des pas fictifs puisque toutes les spires du ressort sont jointives.

## Revendications

1. Support de guidage (1) pour cathéter comprenant un fil (2) ayant une partie proximale (6) de section sensiblement constante et une partie distale (8) effilée, et une gaine (4) de protection souple formée d'un ressort hélicoïdal s'étendant au moins autour de la longueur de la partie distale (8) effilée, le fil (2) étant rendu solidaire de la gaine (4) par au moins l'extrémité libre de sa partie proximale au moyen d'une première soudure (10) dans une partie proximale (12) de la gaine, l'extrémité libre (14) de la partie distale de la gaine (4) étant munie d'une deuxième soudure (16) formant un bout arrondi, caractérisé en ce que le ressort formant la gaine comprend, sur toute sa longueur, un premier brin (18) roulé avec un premier pas pour former une série de premières spires (S18) et au moins un deuxième brin (20, 22, 24) roulé avec un deuxième pas d'une valeur sensiblement supérieure à celle du premier pas pour former une série de deuxièmes spires (S20, S22, S24), de manière que les premières spires (S18) exercent une force de compression sur les deuxièmes spires (S20, S22, S24)

2. Support de guidage pour cathéter selon la revendication 1, caractérisé en ce que le ressort comprend quatre brins (18, 20, 22, 24).
